# EUROPÄISCHE PATENTSCHRIFT

(11) **EP 2 198 262 B1**
(45) Veröffentlichungstag und Bekanntmachung des Hinweises auf die Patenterteilung: **29.06.2011**
(21) Anmeldenummer: 08802628.1
(22) Anmeldetag: 25.09.2008
(51) Int. Cl.: G01N 3/56, G01N 33/42

(54) **VORRICHTUNG UND VERFAHREN ZUM UNTERSUCHEN DER VERSCHLEISSFESTIGKEIT EINER OBERFLÄCHE EINER STRASSENBELAGSPROBE**
APPARATUS AND METHOD FOR INVESTIGATING THE WEAR RESISTANCE OF A SURFACE OF A ROAD SURFACING SAMPLE
DISPOSITIF ET PROCÉDÉ DE TEST DE LA RÉSISTANCE À L'USURE DE LA SURFACE D'UNE ÉPROUVETTE DE REVÊTEMENT ROUTIER

(30) Priorität: 11.10.2007 DE 102007048839
(43) Veröffentlichungstag der Anmeldung: 23.06.2010
(73) Patentinhaber: Technische Universität Darmstadt, 64289 Darmstadt (DE)
(72) Erfinder: ROOT, Viktor, 64293 Darmstadt (DE); BOTT, Alois, 64407 Fränkisch-Crumbach (DE); SEEHAUS, Rainer, 64291 Darmstadt (DE); BALD, J., Stefan, 64285 Darmstadt (DE)
(74) Vertreter: Schmid, Nils T.F.
(86) Internationale Anmeldenummer: PCT/EP2008/008166
(87) Internationale Veröffentlichungsnummer: WO 2009/049756

(56) Entgegenhaltungen:
- AT-B- 314 874
- JP-A- 10 260 114
- JP-A- 2002 082 033

## Beschreibung

Die Erfindung betrifft eine Vorrichtung und ein Verfahren zum Untersuchen der Verschleißfestigkeit einer Oberfläche einer Straßenbelagsprobe. Um Straßenbeläge hinsichtlich ihrer alltäglichen Belastung vor allem in deren Aufbau und Einsatz von Materialien zu optimieren, ist es geboten, die Oberfläche von Straßenbelägen durch möglichst praxisnahe Simulationen im Labor prüfen zu können.

Eine Vorrichtung zum Untersuchen der Verschleißfestigkeit von Straßenbaustoffen ist aus der österreichischen Patentschrift mit der Nummer 314874 bekannt, bei der ein vertikal verlagerbarer Reifen in einen Lastkontakt mit einer Straßenbelagsprobe kommt. Die Straßenbelagsprobe ist über eine Spannvorrichtung fixiert, welche drehbar gelagert ist. Der Reifen wird über eine Antriebswelle angetrieben.

Die Patentschrift JP10260114 offenbart eine Vorrichtung zum Untersuchen der Verschleißfestigkeit einer Fahrschiene, bei der ein vertikal bewegliches Rad in Lastkontakt mit einer Fahrschiene kommt. Das Rad ist frei drehbar angeordnet. Die Fahrschiene wird in Abrollrichtung des Rads parallel zur Oberfläche bewegt.

Die Erfinder haben herausgefunden, dass schwere Verschleißschäden an Straßenbelägen insbesondere dann hervorgerufen werden, wenn die Straßenbelagsoberfläche Lenkbewegungen von stehenden oder leicht rollenden Reifen eines Kraftfahrzeugs ausgesetzt wird. Insbesondere Kraftfahrzeugfahrer, die Parkvorgänge initiieren oder beispielsweise vor Ampeln oder im Stau stehend Spurwechsel vornehmen wollen, teilen dem Straßenbelag bei Gleitreibung enorme Schubkräfte mit, welche ein Lockern und sogar ein Herauslösen von Bestandteilen aus dem Straßenbelag bewirken können. Üblicherweise bestehen Asphaltdeckschichten aus einem Gemisch aus feinen und groben Gesteinskörnern, einem Füller und Straßenbaubitumen. Jede dieser Komponenten hat bestimmte Aufgaben, um eine Dauerbeständigkeit des Straßenbelags zu verbessern. Das durch Verschleiß verursachte Entfernen nur einer der Komponenten aufgrund von starken Schubkräften kann zu einem schnell sich ausweitenden Verschleiß des gesamten Straßenbelagsbereichs führen.

Mit der oben genannten Verschleißuntersuchungsvorrichtung ist eine Simulation von hohen Schubkräften, wie sie bei Lenkvorgängen von leicht rollenden Rädern auftreten, nicht realisierbar.

Es ist Aufgabe der Erfindung, die Nachteile des Stands der Technik zu überwinden, insbesondere eine Laboruntersuchung zur Bestimmung der Verschleißfestigkeit einer Oberfläche einer Straßenbelagsprobe bereitzustellen, bei der hohe Schubkräfte des Straßenbelags mitgeteilt werden, die im wesentlichen bei Lenkbewegungen von stehenden oder leicht rollenden Rädern auftreten.

Diese Aufgabe wird durch die Merkmale von Anspruch 1 oder 10 gelöst.

Danach ist eine Vorrichtung oder ein Verfahren zum Untersuchen der Verschleißfestigkeit eines Oberflächenbereichs einer Straßenbelagsprobe vorgesehen, die eine schwenkbare Aufnahme zum Fixieren der Straßenbelagsprobe aufweist. Vorzugsweise ist die Aufnahme horizontal ausgerichtet, so dass eine freie Oberfläche der Straßenbelagsprobe in einer horizontalen Ebene liegt. Des Weiteren umfasst die Untersuchungsvorrichtung eine Tragachse, an der ein Rad insbesondere frei drehbar angeordnet ist. Des Weiteren hat die Untersuchungsvorrichtung einen Stellantrieb, um die Oberfläche der Straßenbelagsprobe mit dem Rad zu belasten oder zu entlasten. Dabei kann der Stellantrieb das Rad vertikal relativ zur Oberfläche der Straßenbelagsprobe verlagern. Zudem umfasst die Untersuchungsvorrichtung einen Schwenkantrieb zum Schwenken der Aufnahme um eine die Straßenbelagsprobe lotrecht durchsetzende Schwenkachse. Mit dem Schwenken der Aufnahme und der damit kombinierten Drucklast des auf der Straßenbelagsprobe lastenden Rads können die Schubkräfte erzeugt werden, die bei Lenkvorgängen von stehenden Rädern mitgeteilt werden. Erfindungsgemäß ist die Untersuchungsvorrichtung zusätzlich mit einem Linearantrieb versehen, der die Aufnahme in eine Abrollrichtung des Rads gemäß dessen Ausrichtung parallel zur Oberfläche verlagert. Der Linearantrieb kann die Aufnahme in horizontaler Richtung verlagern, um damit kombiniert mit der Schwenkbewegung der Aufnahme und der Drucklast des Rades eine Lenkbewegung eines leicht rollenden Rades realitätsgetreu nachzuahmen.

Es zeigte sich, dass das erfindungsgemäße Verfahren und die erfindungsgemäße Vorrichtung ausgesprochen praxisnahe Bedingungen bei der Simulierung von Lenkbewegungen von abrollenden Rädern erzeugen können, weil laborseitig die gleichen Verschleißerscheinungen wahrgenommen werden konnten, die häufig auf Parkplätzen und vor Ampeln auftreten. Mit der Erfindung können nunmehr Spezialstraßenbeläge entwickelt und ausreichend fundiert vor dem tatsächlichen Straßeneinsatz überprüft werden, die besonders häufig Lenkbewegungen von stehenden und leicht rollenden Rädern ausgesetzt sind.

Bei einer bevorzugten Ausführung der Erfindung ist die Tragachse des Rads in horizontaler Richtung ortsfest gelagert. Somit wird die Abrollbewegung auf der Straßenbelagsprobe ausschließlich durch den Linearbetrieb realisiert, der die Aufnahme linear verlagert.

Vorzugsweise ist das Rad derart zur Straßenbelagsprobe ausgerichtet, dass dessen Kontaktstelle oder Abrollfläche an der Oberfläche der Straßenbelagsprobe bei einer vollständigen Hin- oder Herbewegung der Aufnahme einmal von der Schwenkachse gekreuzt wird. Mit dieser optimierten Ausrichtung des Rads ist gewährleistet, dass die typischen Schubkräfte von stehenden und rollenden Lenkrädern der Straßenbelagsprobe mitgeteilt werden.

Bei einer bevorzugten Ausführung der Erfindung hat der Linearantrieb einen hin- und her bewegbaren Träger, an dem die schwenkbare Aufnahme ortsfest gelagert ist. Vorzugsweise ist an dem Träger eine Kraftmaschine, wie ein Elektromotor, fest angebracht, welche die Aufnahme schwenkt. Dabei kann die Aufnahme mit der Kraftmaschine über ein Getriebe, insbesondere ein Stirnradgetriebe, vorzugsweise mit einer Untersetzung gekoppelt sein.

Bei einer Weiterbildung der Erfindung sind an einer im wesentlichen freien Oberseite des plattenartigen Trägers die Aufnahme und die Kraftmaschine angeordnet, wobei sich die Antriebswellen der Aufnahme und der Kraftmaschine jeweils durch den Träger hindurch erstrecken, die durch das Untersetzungsgetriebe einer Unterseite des Trägers zugewandt miteinander verbunden sind.

Vorzugsweise ist der Träger an einem ortsfesten Gestell durch einen horizontalen Schlitten linear geführt. Das Untersetzungsgestell kann insbesondere zwischen dem Schlitten und dem Träger angeordnet sein. Bei einer Weiterbildung der Erfindung ist der Träger von einem Motor, insbesondere von einem Elektromotor, betrieben, der mit dem Träger über eine Schubkurbel oder eine Kreuzschleife gekuppelt ist.

Bei einer bevorzugten Ausführung der Erfindung kann der Motor für den Linearantrieb und der Motor für den Schwenkantrieb durch eine einzige Kraftmaschine gebildet sein, die über entsprechende Getriebe die gewünschte Dreh- und Linearbewegung veranlassen kann.

Bei einer Weiterbildung der Erfindung kann der Stellantrieb die Tragachse vertikal relativ zur Aufnahme verlagern. Dabei kann insbesondere die Aufnahme in vertikaler Richtung ortsfest gelagert sein.

Vorzugsweise hat die Aufnahme eine Spanneinrichtung zum Befestigen der Straßenbelagsprobe, welche Spanneinrichtung durch wenigstens eine Spannbacke gebildet ist. Vorzugsweise hat die Spannbacke eine Stellschraube.

Des Weiteren kann vorzugsweise die Aufnahme mit einer Erwärmungseinrichtung versehen sein, die insbesondere durch eine elektrische Heizmatte gebildet ist.

Vorzugsweise ist die Aufnahme zum Halten einer etwa 26 cm mal 26 cm abgemessenen Straßenbelagsprobe dimensioniert.

Bei einer Weiterbildung der Erfindung ist der Schwenkantrieb für eine oszillierende Schwenkbewegung ausgelegt. Dabei kann eine Schwenkamplitude auf bis zu 180° begrenzt sein.

Vorzugsweise ist der Linearantrieb für eine oszillierende Hin- und Herbewegung ausgelegt, wobei eine Bewegungsamplitude auf wenigstens einen halben, vorzugsweise wenigstens einen ganzen, Rollumfang des Rads begrenzt ist.

Bei einer Weiterbildung der Erfindung ist eine Steuerung und/oder Regelung zum Einstellen von Untersuchungsparametern, wie eine Vertikallast des Rads, eine Drehgeschwindigkeit der Aufnahme, einer Lineargeschwindigkeit der Aufnahme, eine Temperatur der Straßenbelagsprobe, eine Anzahl der Untersuchungszahlen, wie Schwenk- und Linearverlagerungszyklen, oder dergleichen, vorgesehen.

Des weiteren betrifft die Erfindung das Verfahren zur Untersuchung der Verschleißfestigkeit einer Straßenbelagsprobe, wobei eine Straßenbelagsprobe in einer Aufnahme fixiert wird, die Straßenbelagsprobe durch das Rad be- und entlastet wird, und die Aufnahme bei der durch das Rad belasteten Straßenbelagsprobe um eine die Straßenbelagsprobe durchsetzende Schwenkachse geschwenkt wird. Erfindungsgemäß wird beim Schwenken der Aufnahme letztere relativ zum Rad linear in deren Rollrichtung verlagert.

Vorzugsweise wird eine Vertikallast des Rads von bis zu 4000 N, eine Drehgeschwindigkeit der Aufnahme von etwa bis 40 1/min, eine Lineargeschwindigkeit der Aufnahme von etwa bis zu 0,3 m/s und/oder eine Temperatur der Straßenbelagsprobe von etwa 20 bis 60° eingestellt.

Um die Verschleißschäden an der Straßenbelagsprobe festzustellen, wird die Masse der Straßenbelagsprobe vor und nach dem Untersuchungsverfahren bestimmt und daraus eine Differenz gebildet.

Vorzugsweise ist die Tragachse in einer Ausgangsstellung des Verfahrens derart zur Schwenkachse ausgerichtet, dass die Schwenkachse im Wesentlichen die Tragachse kreuzt.

Es sei klar, dass das erfindungsgemäße Verfahren entsprechend der Funktionsweise der erfindungsgemäßen Vorrichtung verfahren kann.

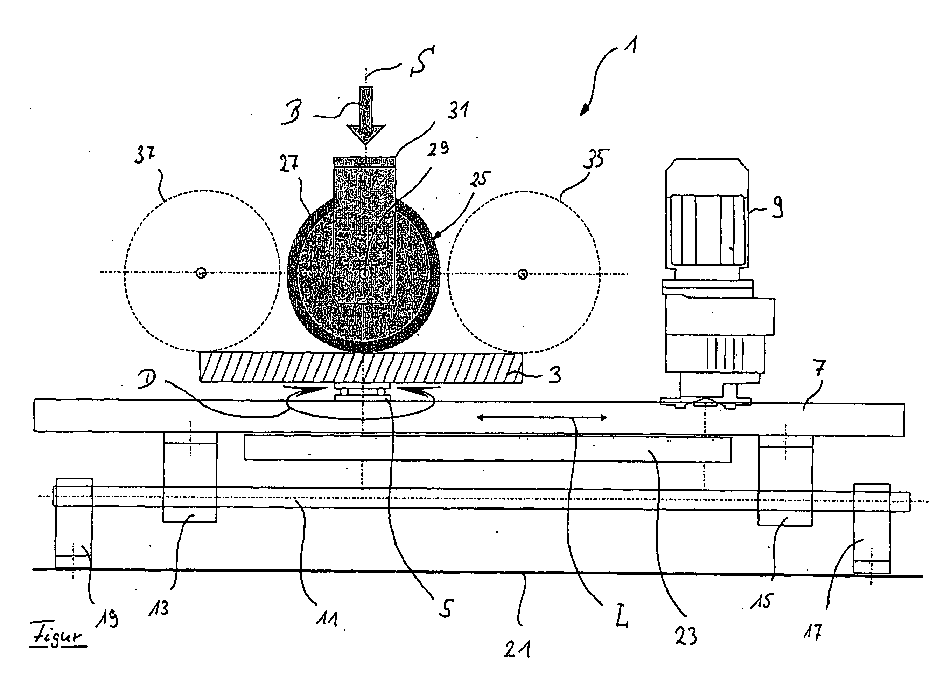

Weitere Vorteile, Merkmale und Eigenschaften der Erfindung werden durch die folgende Beschreibung einer bevorzugten Ausführung der Erfindung anhand der beiliegenden Zeichnung deutlich, in der eine Figur eine schematische Querschnittsansicht einer erfindungsgemäßen Prüfvorrichtung zeigt.

In der Figur ist die erfindungsgemäße Prüfvorrichtung im Allgemeinen mit der Bezugsziffer 1 versehen.

Die Prüfvorrichtung 1 hat eine Aufnahme 3 für eine 26 cm mal 26 cm große Straßenbelagsprobe (nicht näher dargestellt), die mittels einer nicht dargestellten Einspannvorrichtung mit Stellschrauben befestigt ist. Damit keine Innenspannungen in der Straßenbelagsprobe entstehen können, sind die Stellschrauben manuell zu bedienen.

Die Aufnahme 3 ist um eine vertikale Schwenkachse S drehbar gelagert, was durch den gekrümmten Doppelpfeil D angedeutet sein soll. Für die drehbare Lagerung der Aufnahme 3 ist ein Kugellager 5 repräsentativ angedeutet, das die Aufnahme 3 mit einem plattenförmigen Träger 7 koppelt.

Der Träger 7 ist Teil eines Linearantriebs. Der Träger 7 ist als Platte ausgeführt, an deren Oberseite neben der Aufnahme 3 ein Drehstrommotor 9 fest angeordnet ist, der mit einem nicht näher dargestellten Stirnantrieb versehen ist.

Der Träger 7 ist in horizontaler Richtung hin und her verlagerbar, was durch den geraden Doppelpfeil L angedeutet sein soll. Um eine definierte Hin- und Herbewegung des Trägers 7 zu gewährleisten, ist der Träger 7 an einer Schiene 11 geführt, entlang welcher der Träger über ein Paar Schlitten 13, 15 verschieblich ist. Die Schiene 11 ist über ein Gestell 17, 19 mit einem Fundament 21 fest verbunden.

Zwischen dem Träger 7 und der Schiene 11 ist ein Untersetzungsgetriebe 23 angeordnet, das das Stirnantriebsrad des Drehstrommotors 9 mit einer Antriebswelle (nicht näher dargestellt) der Aufnahme 3 untersetzend kuppelt.

Ein Prüfrad 25 mit einem Reifen 27 ist an einer Tragachse 29 frei drehbar gelagert, welche von einem Gabelträger 31 gehalten wird. Der Gabelträger 31 wird von einem pneumatischen Stellantrieb (nicht näher dargestellt) in vertikaler Richtung verlagert, um die Straßenbelagsprobe durch das Prüfrad 25 vertikal zu belasten, was mit dem breiten Pfeil B angedeutet sein soll. Der pneumatische Stellantrieb dient auch dazu, das Prüfrad von der Straßenbelagsprobe zu entfernen.

Die beidseits gestrichelten Umfänge 35, 37 des Prüfrads 25 sollen lediglich die Position des Prüfrads relativ zur Straßenbelagsprobe andeuten, wenn die Aufnahme 3 zusammen mit dem Träger 7 hin- und her bewegt wird (L). Es sei klar, dass das Prüfrad 25, insbesondere deren Lagerung, in horizontaler Richtung ortsfest ist. Die Rollbewegung des Prüfrads 25 auf den Straßenbelag soll ausschließlich durch den Linearantrieb des Trägers 7 bereitgestellt sein.

Bei einem kombiniert angesteuerten Schwenk- und Linearantrieb kann die in der Aufnahme 3 fixierte Straßenbelagsprobe mit einer Schubbelastung beaufschlagt werden, die typisch für eine Lenkbewegung bei einem leicht rollenden Kraftfahrzeugrad ist.

Es sei klar, dass der Drehstrommotor 9 nicht nur zum Schwenkantrieb der Aufnahme, sondern auch zum Linearantrieb des Trägers 7 eingesetzt werden kann, indem entsprechend eine kuppelnde Kreuzschleife eingesetzt wird.

Die in der vorstehenden Beschreibung, den Figuren und den Ansprüchen offenbarten Merkmale können sowohl einzeln als auch in beliebiger Kombination für die Realisierung der Erfindung in den verschiedenen Ausgestaltungen von Bedeutung sein.

### Bezugszeichenliste

- 1: Prüfvorrichtung
- 3: Aufnahme
- 5: Kugellager
- 7: Träger
- 9: Drehstrommotor
- 11: Schiene
- 13, 15: Schlitten
- 17, 19: Gestell
- 21: Fundament
- 23: Untersetzungsgetriebe
- 25: Prüfrad
- 27: Reifen
- 29: Tragachse
- 31: Gabelträger
- 35, 37: Umfänge
- B: Pfeil
- D, L: Doppelpfeil
- S: Schwenkachse

## Patentansprüche

1. Vorrichtung zum Untersuchen der Verschleißfestigkeit einer Straßenbelagsprobe, umfassend eine schwenkbare Aufnahme (3) zum Fixieren der Straßenbelagsprobe, eine Tragachse (29), an der ein Rad (25) drehbar angeordnet ist, einen Stellantrieb zum Be- und Entlasten einer Oberfläche der Straßenbelagsprobe mit dem Rad (25), einen Schwenkantrieb zum Schwenken der Aufnahme (3) um eine die Straßenbelagsprobe lotrecht durchsetzende Schwenkachse (S), **gekennzeichnet durch** einen Linearantrieb, der die Aufnahme (3) in Abrollrichtung des Rads (25) parallel zur Oberfläche verlagert.

2. Vorrichtung nach Anspruch 1, **dadurch gekennzeichnet, dass** die Tragachse (29) des Rads (25) in horizontaler Richtung ortsfest gelagert ist.

3. Vorrichtung nach Anspruch 1 oder 2, **dadurch gekennzeichnet, dass** das Rad (25) derart zur Straßenbelagsprobe ausgerichtet ist, dass dessen Kontaktstelle oder Abrollfläche an der Oberfläche der Straßenbelagsprobe bei einer vollständigen Hin- oder Herbewegung der Aufnahme (3) einmal von der Schwenkachse (S) gekreuzt wird.

4. Vorrichtung nach einem der Ansprüche 1 bis 3, **dadurch gekennzeichnet, dass** der Linearantrieb einen hin und her bewegbaren Träger (7) aufweist, an dem die Aufnahme (3) ortsfest gelagert ist, wobei insbesondere an dem Träger (7) eine Kraftmaschine, wie ein Elektromotor, fest angebracht ist, die die Aufnahme (3) schwenkt, wobei insbesondere die Aufnahme (3) mit der Kraftmaschine über einen Getriebe, insbesondere Stirnradgetriebe, gekuppelt ist, wobei insbesondere an einer Oberseite des plattenartigen Trägers (7) die Aufnahme (3) und die Kraftmaschine angeordnet sind, wobei sich Antriebswellen der Aufnahme (3) und der Kraftmaschine durch den
Träger (7) hindurch erstrecken, die über ein Untersetzungsgetriebe auf einer Unterseite des Trägers (7) miteinander verbunden sind.

5. Vorrichtung nach Anspruch 4, **dadurch gekennzeichnet, dass** der Träger (7) an einem ortsfesten Gestell durch einen Schlitten (13, 15) linear geführt ist, wobei insbesondere das Untersetzungsgetriebe zwischen dem Schlitten (13, 15) und dem Träger (7) angeordnet ist, und/oder der Träger (7) von einem Motor, insbesondere von einem Elektromotor, betrieben ist, der mit dem Träger (7) über eine Schubkurbel oder einer Kreuzschleife gekuppelt ist.

6. Vorrichtung nach einem der Ansprüche 1 bis 5, **dadurch gekennzeichnet, dass** der Stellantrieb die Tragachse (29) vertikal relativ zur Aufnahme (3) verlagern kann, wobei insbesondere die Aufnahme (3) in vertikaler Richtung ortsfest gelagert ist, und/oder die Aufnahme (3) eine Spanneinrichtung zum Befestigen der Straßenbelagsprobe aufweist, welche Spanneinrichtung durch wenigstens eine Spannbacke gebildet ist.

7. Vorrichtung nach einem der Ansprüche 1 bis 6, **dadurch gekennzeichnet, dass** die Aufnahme (3) mit einer Erwärmeinrichtung versehen ist, die insbesondere durch eine elektrische Heizmatte gebildet ist, und/oder die Aufnahme (3) zum Halten einer etwa 26 cm mal 26 cm abgemessenen Straßenbelagsprobe dimensioniert ist.

8. Vorrichtung nach einem der Ansprüche 1 bis 7, **dadurch gekennzeichnet, dass** der Schwenkantrieb für eine oszillierende Schwenkbewegung ausgelegt ist und eine Schwenkamplitude von bis zu 180° aufweist und/oder der Linearantrieb für eine oszillierende Hin- und Herbewegung ausgelegt ist und eine Bewegungsamplitude von wenigstens einem halben, vorzugsweise wenigstens einem ganzen, Rollumfang des Rades (25) aufweist.

9. Vorrichtung nach einem der Ansprüche 1 bis 8, **dadurch gekennzeichnet, dass** eine Steuerung und/oder Regelung zum Einstelle, von Untersuchungsparame tem, wie eine Vertikallast des Rads (25), eine Drehgeschwindigkeit der Aufnahme, eine Lineargeschwindigkeit der Aufnahme, eine Temperatur der Straßenbelagsprobe, eine Anzahl der Untersuchungszyklen, wie Schwenk- und Linearverlagerungszyklen, oder dergleichen, vorgesehen ist.

10. Verfahren zum Untersuchen der Verschleißfestigkeit einer Oberfläche einer Straßenbelagsprobe, wobei eine Straßenbelagsprobe in einer Aufnahme (3) fixiert wird, die Straßenbelagsprobe durch ein Rad be- und entlastet wird und die Aufnahme (3) bei durch das Rad (25) belasteter Straßenbelagsprobe um eine die Straßenbelagsprobe durchsetzende Schwenkachse (S) geschwenkt wird, **dadurch gekennzeichnet, dass** bei Schwenken die Aufnahme (3) relativ zum Rad linear verlagert wird.

11. Verfahren nach Anspruch 10, **dadurch gekennzeichnet, dass** die Straßenbelagsprobe oszillierend um etwa180° geschwenkt wird.

12. Verfahren nach Anspruch 10 oder 11, **dadurch gekennzeichnet, dass** eine Vertikallast des Rads (25) von bis zu 4000 N, eine Drehgeschwindigkeit der Aufnahme (3) von etwa bis 40 1/min, eine Lineargeschwindigkeit der Aufnahme (3) von etwa bis zu 0,3 m/s und/oder eine Temperatur der Straßenbelagsprobe von etwa 20 bis 60°C eingestellt wird.

13. Verfahren nach einem der Ansprüche 10 bis 12, **dadurch gekennzeichnet, dass** die Masse der Straßenbelagsprobe vor und nach dem Untersuchungsverfahren bestimmt wird und eine Differenz gebildet wird.

14. Verfahren nach einem der Ansprüche 10 bis 13, **dadurch gekennzeichnet, dass** in einer Ausgangsstellung die Tragachse (29) des Rads (25) derart zur Schwenkachse ausgerichtet wird, dass die Schwenkachse (S) im wesentlichen die Tragachse (29) kreuzt.

15. Verfahren nach einem der Ansprüche 10 bis 14, **dadurch gekennzeichnet, dass** entsprechend der Funktionsweise der nach einem der Ansprüche ausgebildeten Vorrichtung verfahren wird.

## Claims

1. Apparatus for investigating the wear resistance of a road surface sample, comprising a pivotable receiving organ (3) for fixating the road surface sample, a support axis (29) at which a wheel (25) is rotatably disposed, a positioning drive for loading and unloading a surface of the road surface sample with the wheel (25), a pivot drive for pivoting the receiving organ (3) around a pivot axis (S) which passes vertically through the road surface sample, **characterised by** a linear drive displacing the receiving organ (3) in rolling direction of the wheel (25) and parallel to the surface.

2. Apparatus according to claim 1, **characterised in that** the support axis (29), of the wheel (25) is mounted stationarily in horizontal direction.

3. Apparatus according to claim 1 or 2, **characterised in that** the wheel (25) is oriented with respect to the road surface sample in such a way that its contact point or rolling surface is crossed once by the pivot axis (S) for a complete back or forth movement of the receiving organ (3).

4. Apparatus according to one of the claims 1 to 3, **characterised in that** the linear drive features a support (7), movable back and forth, at which the receiving organ (3) is mounted in a fix position, wherein, in particular at the support (7), a power machine such as an electric motor is firmly attached, pivoting the receiving organ (3), wherein in particular the receiving organ (3) is coupled to the power machine via a gear unit, in particular a spur gear transmission, wherein the receiving organ (3) and the power machine are disposed in particular at an upper side of the plate shaped support (7), wherein drive shafts of the receiving organ (3) and the power machine extend through the support (7) and are connected to each other at a lower side of the support (7) through a reduction gear.

5. Apparatus according to claim 4, **characterised in that** the support (7) is linearly guided at a stationary rack by means of a slider (13, 15), wherein in particular the reduction gear is disposed between the slider (13, 15) and the support (7), and/or the support (7) is driven by a motor, in particular by an electric motor coupled to the support (7) via a slider crank or a scotch yoke.

6. Apparatus according to one of the claims 1 to 5, **characterised in that** the positioning drive can displace the support axis (29) vertically with respect to the receiving organ (3), wherein in particular the receiving organ (3) is stationarily mounted in vertical direction and/or the receiving organ (3) presents a clamping device for fixing the road surface sample, the clamping device being formed by at least one clamping jaw.

7. Apparatus according to one of the claims 1 to 6, **characterised in that** the receiving organ (3) is provided with a heating device, in particular formed by an electric heating mat, and/or the receiving organ (3) is dimensioned to hold a road surface sample measuring about 26 cm by 26 cm.

8. Apparatus according to one of the claims 1 to 7, **characterised in that** the pivot drive is designed for an oscillating pivoting movement and features a pivoting amplitude of up 180°, and/or the linear drive is designed for an oscillating back and forth movement and features an amplitude of movement of at least half, preferably at least a full rolling circumference of the wheel (25).

9. Apparatus according to one of the claims 1 to 8, **characterised in that** a control device is provided for the adjustment of investigation parameters such as a vertical load of the wheel (25), a rotational speed of the receiving organ, a linear speed of the receiving organ, a temperature of the road surface sample, a number of investigation cycles such as pivot and linear displacement cycles, or the like.

10. Method for investigating the wear resistance of a road surface sample, wherein a road surface sample is fixated in a receiving organ (3), the road surface sample is loaded and unloaded by a wheel, and while the road surface sample is loaded by the wheel the receiving organ (3) is pivoted around a pivot axis (S) passing through the road surface sample (25), **characterised in that** during pivoting the receiving organ (3) is linearly displaced with respect to the wheel.

11. Method according to claim 10, **characterised in that** the road surface sample is oscillatingly pivoted by about 180°.

12. Method according to claim 10 or 11, **characterised in that** a vertical load of the wheel (25) of up to 4000 N, a rotational speed of the receiving organ (3) of about up to 40 1/min, a linear speed of the receiving organ (3) of about up to 0.3 m/s and/or a temperature of the road surface sample of about 20 to 60°C is adjusted.

13. Method according to one of the claims 10 to 12, **characterised in that** the mass of the road surface sample is determined before and after the investigation and a difference is calculated.

14. Method according to one of the claims 10 or 13,**characterised in that** in an initial position the support axis (29) of the wheel (25) is oriented with respect to the pivot axis such that the pivot axis (S) essentially crosses the support axis (29).

15. Method according to one of the claims 10 or 14, **characterised in that** it proceeds according to the functionality of the apparatus as in one of the claims.

## Revendications

1. Dispositif pour examiner la résistance à l'usure d'un échantillon de revêtement routier, comprenant un organe récepteur (3) pour immobiliser l'échantillon de revêtement routier, un axe de support (29) auquel une roue (25) est agencée de façon pivotable, un entraînement de réglage pour charger et décharger une surface de l'échantillon de revêtement routier par la roue (25), un entraînement en pivotement pour faire pivoter l'organe récepteur (3) autour d'un axe de pivotement (S) qui passe verticalement à travers l'échantillon de revêtement routier, **caractérisé par** un entraînement linéaire qui déplace l'organe récepteur (3) en direction de roulement de la roue (25) et en parallèle à la surface.

2. Dispositif selon la revendication 1, **caractérisé en ce que** l'axe de support (29) de la roue (25) est montée de façon immobile en direction horizontale.

3. Dispositif selon la revendication 1 ou 2, **caractérisé en ce que** la roue (25) est orientée par rapport à l'échantillon de revêtement routier de telle façon que sa zone de contact ou surface de roulement à la surface de l'échantillon de revêtement routier est croisé par l'axe de pivotement (S) une fois pour un mouvement complet aller ou retour de l'organe récepteur (3).

4. Dispositif selon l'une des revendications 1 à 3, **caractérisé en ce que** l'entraînement linéaire comprend un support (7), mobile en mouvement alternatif, auquel l'organe récepteur (3) est monté à un endroit fixe, une machine à effort, tel qu'un moteur électrique, qui fait pivoter l'organe récepteur (3) étant solidement fixée au support (7), dans lequel l'organe récepteur (3) est en particulier couplé avec la machine à effort à travers d'un engrenage, en particulier un engrenage droit, l'organe récepteur (3) et la machine à effort étant disposés en particulier à la face supérieure du support (7) en forme de plaque, des arbres d'entraînement de l'organe récepteur (3) et de la machine à effort passant à travers le support (7) et étant liés entre eux par un engrenage de réduction à une face inférieure du support (7).

5. Dispositif selon la revendication 4, **caractérisé en ce que** le support (7) est guidé linéairement sur un cadre par un coulisseau, dans lequel en particulier l'engrenage de réduction est disposé entre le coulisseau (13, 15) et le support (7), et/ou le support (7) est entraîné par un moteur, en particulier un moteur électrique couplé avec le support (7) par une manivelle de poussée ou un excentrique Scotch.

6. Dispositif selon l'une des revendications 1 à 5, **caractérisé en ce que** l'entraînement de réglage est apte à déplacer verticalement l'axe de support (29) relatif à l'organe récepteur (3), qui, en particulier, est monté de façon fixe en direction verticale, et/ou l'organe récepteur (3) présente un dispositif de serrage pour fixer l'échantillon de revêtement routier, le dispositif de serrage étant formé par au moins une mâchoire de serrage.

7. Dispositif selon l'une des revendications 1 à 6, **caractérisé en ce que** l'organe récepteur (3) est muni d'un dispositif de chauffage étant formé en particulier par un tapis chauffant électrique, et/ou l'organe récepteur (3) est dimensionné pour tenir un échantillon de revêtement routier mesurant environs 26 cm sur 26 cm.

8. Dispositif selon l'une des revendications 1 à 7, **caractérisé en ce que** l'entraînement en pivotement est conçu pour un mouvement pivotant oscillatoire et présente une amplitude de pivotement de jusqu'à 180° et/ou l'entraînement linéaire est conçu pour un mouvement alternatif oscillatoire et présente une amplitude de mouvement d'au moins une demie, préférablement une entière circonférence de rotation de la roue (25).

9. Dispositif selon l'une des revendications 1 à 8, **caractérisé en ce qu'**un contrôle et/ou réglage est pourvu pour ajuster des paramètres d'examen, tel qu'une charge verticale de la roue (25), une vitesse de rotation de l'organe récepteur (3), une vitesse linéaire de l'organe récepteur (3), une température de l'échantillon de revêtement routier, un nombre des cycles d'examen, tel que des cycles de pivotement ou de déplacement linéaire, ou d'autres.

10. Procédé pour examiner la résistance à l'usure d'un échantillon de revêtement routier, dans lequel un échantillon de revêtement routier est fixé dans un organe récepteur (3), l'échantillon de revêtement routier est chargé et déchargé par une roue, et l'organe récepteur (3) pivote autour d'un axe de pivotement (S) passant à travers l'échantillon de revêtement routier, l'échantillon de revêtement routier étant sous charge par la roue, **caractérisé en ce que** l'organe récepteur (3) est déplacé relatif à la roue pendant le pivotement.

11. Procédé selon la revendication 10, **caractérisé en ce que** l'échantillon de revêtement routier est pivoté par environs 180°.

12. Procédé selon la revendication 10 ou 11, **caractérisé en ce qu'**un effort vertical de la roue (25) de jusqu'à 4000N, une vitesse rotative de l'organe récepteur (3) d'environs jusqu'à 40 1/min, une vitesse linéaire de l'organe récepteur (3) d'environs jusqu'à 0,3 m/s et/ou une température de l'échantillon de revêtement routier d'environs 20 à 60°C est ajusté.

13. Procédé selon l'une des revendications 10 à 12, **caractérisé en ce que** la masse de l'échantillon de revêtement routier est déterminée avant et après l'examen et qu'une différence est calculée.

14. Procédé selon l'une des revendications 10 à 13, **caractérisé en ce que** dans une position de départ l'axe de support (29) de la roue (25) est orienté par rapport à l'axe de pivotement tel que l'axe de pivotement (S) sensiblement croise l'axe de support (29).

15. Procédé selon l'une des revendications 10 à 14, **caractérisé en ce que** l'on procède d'après la fonctionnalité du dispositif selon l'une des revendications.
